# FASCICULE DE BREVET EUROPEEN

(11) **EP 1 901 716 B1**
(45) Date de publication et mention de la délivrance du brevet: **04.07.2018**
(21) Numéro de dépôt: 06778700.2
(22) Date de dépôt: 28.06.2006
(51) Int. Cl.: A61K 9/16, A61K 9/20, A23G 3/34, A23G 3/42, A23G 3/50, A23L 27/30, A23L 29/30, B01J 2/12

(54) **PROCÉDÉ DE PRODUCTION DE GRANULÉS CONTENANT DES PARTICULES CRISTALLINES DE XYLITOL AVEC UN AUTRE POLYOL**
VERFAHREN ZUR HERSTELLUNG EINES GRANULATS MIT XYLITKRISTALLPARTIKELN MIT EINEM ANDEREN POLYOL
METHOD FOR PRODUCING GRANULES CONTAINING XYLITOL CRYSTAL PARTICLES WITH ANOTHER POLYOL

(30) Priorité: 08.07.2005 FR 0507327
(43) Date de publication de la demande: 26.03.2008
(73) Titulaire: Roquette Frères, 62136 Lestrem (FR)
(72) Inventeur: DUFLOT, Pierrick, 62136 La Couture (FR); BOIT, Baptiste, 62400 Bethune (FR)
(74) Mandataire: Cabinet Plasseraud
(86) Numéro de dépôt international: PCT/FR2006/001507
(87) Numéro de publication internationale: WO 2007/006885

(56) Documents cités:
- EP-A- 0 528 604
- EP-A- 1 072 578
- EP-A- 1 207 164
- WO-A-96/07331
- WO-A-2004/005227
- WO-A-2004/106273
- FR-A- 2 202 867
- US-A- 5 679 398

## Description

L'invention concerne un procédé de fabrication continue de granulés contenant des particules cristallines de xylitol avec un autre polyol en plus faible proportion.

Par « autre polyol », on entend au sens de l'invention un polyol choisi préférentiellement dans le groupe constitué par le threitol, l'érythritol, l'arabitol, le ribitol, le sorbitol, le mannitol, le maltitol, le maltotriitol, le maltotétraitol, le lactitol, l'isomaltul.ose hydrogéné, la glycérine et les hydrolysats d'amidon hydrogénés, pris seuls ou en combinaison.

Plus préférentiellement, cet autre polyol est choisi dans le groupe constitué par le sorbitol, le maltitol, le mannitol et l'isomaltulose hydrogéné, pris seuls ou en combinaison.

Par « plus faible proportion », on entend au sens de l'invention une teneur en cet autre polyol, dans les granulés contenant des particules cristallines de xylitol, comprise entre 2,5 et 15 % en poids, de préférence de 5 à 10 % en poids de la quantité totale de polyols.

Le xylitol est un polyol à 5 atomes de carbone, résultant de l'hydrogénation du xylose. Il est utilisé en tant que substance de substitution du sucre et/ou en tant que substance support dans des préparations pharmaceutiques et dans l'industrie alimentaire, en particulier sous forme de comprimés à sucer ou à mâcher.

On sait déjà comment fabriquer du xylitol cristallisé, par exemple en induisant la cristallisation dudit xylitol dans un sirop suffisamment riche en ce produit et suffisamment purifié. On obtient alors des monocristaux de forme tétraédrique, de taille relativement uniforme.

Cependant, le xylitol pur présente de très mauvaises propriétés de compression, indépendamment du type de fabrication mis en oeuvre.

Afin d'améliorer celles-ci, il est connu dans l'état de la technique de proposer de fabriquer des poudres de xylitol présentant une teneur en xylitol de plus de 90 % en poids, le complément de la teneur totale en polyol étant apporté par un autre polyol, tel le sorbitol.

Il a été proposé dans le brevet EP 528604 de réaliser la co-cristallisation du xylitol et du sorbitol, le rapport entre sorbitol et xylitol étant compris entre 50/50 à 97/3, de préférence compris entre 65/35 et 95/5.

Outre le fait que ces co-cristaux renferment plus de 50 % de sorbitol (le xylitol est ici plutôt le composant minoritaire du mélange), le mélange ne présente en aucun cas des propriétés de compression notables.

Il est également proposé dans le brevet DE 19845339 de réaliser la co-atomisation du xylitol à plus de 90 % en poids avec du sorbitol, ou en granulant ledit mélange dans un lit fluidisé.

Les résultats montrent que le xylitol pur granulé par pulvérisation ne convient pas à une transformation directe en comprimés sans autres additifs. A une pression de 20 kN, la dureté des comprimés, d'environ 60 N, est trop faible et l'abrasion est trop élevée pour les comprimés fabriqués.

Pour les spécialistes du domaine considéré, les duretés ne peuvent en effet être atteintes en pratique que lorsque le xylitol est granulé avec de la carboxyméthylcellulose (CMC) à raison par exemple de 2 % en poids.

La demande de brevet internationale WO 92/10168 décrit également un procédé de fabrication de granulés de xylitol directement compressible consistant en la granulation humide de xylitol finement broyé par l'emploi d'un liant pouvant être une solution de carboxyméthylcellulose sodique, une solution de polydextrose ou encore un sirop de maltose hydrogéné. Un séchage final est obligatoire. On obtient ainsi des granulés contenant de 0,1 à 5 % de liant.

Toutefois, la CMC contenue dans les comprimés se répercute de manière désavantageuse sur les propriétés organoleptiques.

Il a été également proposé, dans le brevet FR 2.336.123, de fabriquer des tablettes à mâcher à partir de mélange sec de xylitol en quantité de 10 à 80 % en poids et d'un polyol en quantité de 80 à 10 % en poids, par rapport au poids de la tablette. Le polyol peut être le sorbitol, le mannitol ou un mélange de ceux-ci.

Il ressort de la description de cette demande de brevet que le polyol représente au moins 50 % en poids par rapport au xylitol. Ce dernier ne peut donc être considéré comme la composante principale de la poudre à comprimer. Dans ces conditions, il n'est pas possible de bénéficier de manière optimale de tous les avantages propres au xylitol.

En agglomérant du xylitol poudre cristallisé de très fine granulométrie à l'aide d'un sirop de sorbitol sous forte agitation, le brevet EP 329977 propose un agent liant et diluant, utilisable en compression directe, dont les granulés ont une taille comprise entre 0,1 et 1 mm et contenant 94 à 98 % en poids de xylitol, 1 à 5 % en poids de sorbitol, 0 à 2 % en poids d'autres polyols, et moins de 1 % en poids d'eau.

Cet agent liant et diluant présente une masse volumique apparente du produit tassé de 0,7 à 0,8 g/ml.

Le broyage préalable du xylitol pour obtenir une fine granulométrie, l'agglomération à l'aide d'un sirop de sorbitol à faible matière sèche, ainsi que le séchage final de la poudre ne donnent pas au procédé toute la simplicité souhaitée et constituent des opérations supplémentaires qui augmentent son coût de mise en oeuvre.

La présente invention a donc comme but particulier de fournir un procédé de fabrication de granulés contenant des particules cristallines de xylitol avec un autre polyol qui sont moins sensibles aux désavantages susmentionnés et qui permet d'obtenir efficacement une poudre contenant des particules cristallines de xylitol ayant des propriétés désirables.

Selon la présente invention, on fournit un procédé de fabrication de particules cristallines de xylitol avec un autre polyol qui ne nécessite pas une très forte concentration de xylitol ou aucun effort entrepris pour contrôler ou mesurer précisément la température pendant l'étape de granulation / cristallisation.

De plus, le procédé de l'invention n'implique pas de formation de masse cuite, ni l'application d'une force de cisaillement ou de pétrissage, reposant plutôt simplement sur un revêtement, une agglomération et une induction de la cristallisation concurrents en permettant au mélange aggloméré de xylitol avec un autre polyol de maturer à une température inférieure au point de fusion du xylitol, pour former des granulés solides.

Selon la présente invention, on fournit un procédé de fabrication de granulés contenant des particules cristallines de xylitol et d'un autre polyol, comprenant le mélange continu d'un sirop de xylitol et d'un autre polyol, présentant une teneur en matière sèche d'au moins 95 % en poids, ayant une teneur en xylitol de 85 a 97,5 % en poids et en l'autre polyol de 15 a 2,5 % en poids sur la base de la matière sèche, ledit mélange étant effectué en dispersant simultanément le sirop de xylitol et de l'autre polyol avec des germes contenant du xylitol dans un récipient rotatif ouvert contenant des granulés à base de xylitol, ce par quoi le sirop de xylitol et de l'autre polyol et les germes contenant du xylitol sont mélangés à la surface des granulés à base de xylitol contenus dans le récipient, la récupération des granulés à base de xylitol et de l'autre polyol ayant un diamètre de 100 a 10.000 µm depuis le récipient, et la cristallisation du xylitol et de l'autre polyol contenus dans lesdits granulés, les granulés à base de xylitol et de l'autre polyol dans le récipient étant maintenus en mouvement par la rotation du récipient. Lors de la réalisation du procédé de l'invention, le sirop de xylitol et de l'autre polyol est introduit de préférence dans le récipient sous une forme subdivisée par exemple sous forme de gouttes ou de globules, de jets ou de faisceaux de jets.

Selon une méthode préférée de réalisation du procédé susmentionné, un sirop de xylitol et de l'autre polyol, présentant une teneur en matière sèche d'au moins 95% en poids, est porté à une température d'au moins 80°C et est mélangé continuellement dans un récipient avec des germes contenant du xylitol, le rapport germes/sirop, les dimensions, l'orientation de l'axe de rotation et la vitesse de rotation du récipient étant choisis de telle façon que le produit récupéré depuis le récipient apparaît sous la forme de granulés d'un diamètre moyen d'environ 100 à 10.000 µm.

Le procédé de l'invention peut être réalisé dans un appareil comprenant un récipient rotatif ouvert, avec un axe de rotation pouvant être incliné horizontalement, un moyen d'apporter, à une zone située à l'intérieur du récipient, au-dessus de la masse qui le remplit partiellement, et d'y disperser une partie de sirop de xylitol et de l'autre polyol, de préférence subdivisé en les formes mentionnées ci-dessus et quelques germes contenant du xylitol, et un moyen d'assurer le mélange du sirop de xylitol et de l'autre polyol et des germes contenant du xylitol, à la surface de la masse en mouvement remplissant partiellement le récipient.

Les granulés à base de xylitol sont récupérés de préférence par débordement à la sortie du récipient et peuvent être maturés pour augmenter leur cristallinité, par le transfert des granulés dans un cylindre rotatif de caractéristiques dimensionnelles telles que la durée du séjour des granulés provenant du récipient est suffisante pour assurer la cristallisation du xylitol et de l'autre polyol. Les granulés peuvent ensuite être transférés dans un séchoir pour diminuer l'humidité résiduelle, et ensuite dans un moyen de broyage et de criblage.

Selon un premier mode préféré de réalisation du procédé de l'invention, on peut se procurer un sirop de xylitol et de sorbitol, du type de celui disponible dans le commerce ou on peut le préparer extemporanément, de manière à ce que sa matière sèche soit d'au moins 95 %, et que sa teneur en xylitol soit d'environ 95 % sur la base de la matière sèche et sa teneur en sorbitol soit d'environ 5 % sur la base de la matière sèche, et on disperse ledit sirop à une température d'environ 80°C à l'intérieur d'un récipient rotatif ouvert sous forme d'un réservoir ou d'un tambour ouvert ayant un fond essentiellement plat, dont l'axe de rotation peut être incliné sur un plan horizontal d'un angle de 25 à 45°.

Selon un deuxième mode préféré de réalisation du procédé de l'invention, on peut préparer extemporanément un sirop de xylitol et de sorbitol dont la matière sèche est d'au moins 95 % et ayant une teneur en xylitol d'environ 85 % sur la base de la matière sèche et en sorbitol d'environ 15 % sur la base de la matière sèche, on disperse ensuite le dit sirop à une température d'environ 80°C à l'intérieur d'un récipient rotatif ouvert sous forme d'un réservoir ou d'un tambour ouvert ayant un fond essentiellement plat, dont l'axe de rotation peut être incliné sur un plan horizontal d'un angle de 25 à 45°.

Selon un troisième mode préféré de réalisation du procédé de l'invention, on prépare extemporanément un sirop de xylitol et de maltitol dont la matière sèche est d'au moins 95 %, et ayant une teneur en xylitol d'environ 90 % sur la base de la matière sèche et en maltitol d'environ 10 % sur la base de la matière sèche, et on disperse ledit sirop à une température d'environ 80°C à l'intérieur d'un récipient rotatif ouvert sous forme d'un réservoir ou d'un tambour ouvert ayant un fond essentiellement plat, dont l'axe de rotation peut être incliné sur un plan horizontal d'un angle de 25 à 45°.

Une buse d'atomisation d'air est avantageusement utilisée, dans ces trois modes préférés de réalisation particuliers pour pulvériser ledit sirop aqueux sur le lit rotatif de matériaux de germes contenant du xylitol, dans un granulateur à échelle pilote.

Le rapport pondéral dans le mélange constitué de germes contenant du xylitol et du sirop de xylitol et de sorbitol (ou de maltitol) est de 1/1.

Le mélange est effectué à la surface de la masse en mouvement remplissant partiellement le récipient ; le mouvement en question rappelle une masse de pilules à l'intérieur d'une machine de fabrication de pilules, et on a constaté que des granulés qui sont de plus en plus gros sont formés, les granulés les plus grands ayant tendance à venir à la surface de la masse en mouvement.

Les granulés de xylitol et de l'autre polyol ainsi obtenus sont ensuite maturés pour augmenter leur cristallinité.

Cette étape de maturation peut être réalisée en conservant les granulés en mouvement à une température inférieure au point de fusion des granulés, préférentiellement à une température de 5 à 85°C, pendant de 1 à 20 heures, dans un courant d'air.

Le produit granulé est ensuite séché afin d'obtenir une humidité résiduelle non supérieure à environ 1 %.

Les granulés peuvent alors être broyés jusqu'à la taille de particules requise et ensuite triés par tamisage ; les particules éliminées par tamisage peuvent être avantageusement recyclées vers le récipient susmentionné, pour une utilisation comme germes contenant du xylitol et l'autre polyol.

Une propriété très avantageuse de la poudre obtenue à partir des granulés contenant des particules cristallines de xylitol et de l'autre polyol préparés par le procédé de l'invention, est qu'elle présente une valeur de compression, selon le test B, qui est supérieure à 80 N, de préférence supérieure à 100 N, plus préférentiellement encore supérieure à 150 N, comme il sera exemplifié ci-après.

Le test B consiste à mesurer la dureté, exprimée en Newtons, d'un échantillon de comprimé produit sur une Presse Carver par une charge de 18 kNewtons appliquée à une matrice annulaire Carver n° 3619 de 13 mm, pour obtenir un comprimé contenant 0,9 g de poudre obtenue à partir des granulés contenant des particules cristallines de xylitol et de l'autre polyol (coupe granulométrique 250 - 425 µm) préparés conforme à l'invention et 1 % de stéarate de magnésium.

La dureté du comprimé est déterminée dans un testeur de comprimés du Dr SCHLEUNIGER PHARMATRON modèle 6D.

Les résultats montrent que la poudre obtenue à partir des granulés préparés conforme à l'invention, contenant des particules cristallines de xylitol et de l'autre polyol, présente une valeur de dureté élevée qui, à la connaissance de la société Demanderesse, n'a jamais encore été décrite.

Les poudres résultantes contenant des particules cristallines de xylitol et de l'autre polyol sont caractérisées également par le fait qu'elles présentent une vitesse de solubilisation dans l'eau, selon le test A, inférieure à 1 minute, de préférence comprise entre 20 et 30 secondes ± 5 secondes.

Afin de mesurer cette caractéristique de la poudre obtenue à partir des granulés contenant des particules cristallines de xylitol et d'un autre polyol préparés selon l'invention, à savoir le temps de solubilisation, on réalise le test A.

La première étape du test A consiste à procéder au dégazage de l'eau à 20°C, eau que l'on place avec un barreau aimanté (longueur 6.06 cm et diamètre 1.15 cm) dans une fiole à vide de 2.000 ml posée sur un agitateur magnétique. La vitesse de rotation du barreau (200 t/mn) est adaptée pour créer une agitation suffisante. La mise sous vide est réalisée jusqu'à disparition de bulles, synonymes de présence résiduelle de gaz dissous.

La seconde étape consiste à tamiser la poudre à tester, de manière à récupérer la fraction présentant une taille de particules comprise entre 315 et 500 µm

On introduit ensuite 150 g d'eau dégazée dans un bécher en verre transparent de forme basse (diamètre extérieur 70 mm, hauteur 96 mm) et de 250 ml, contenant un barreau aimanté cylindrique (45 mm de longueur et 9,5 mm de diamètre).

On place ledit bécher tel que préparé sur un agitateur magnétique de laboratoire réglé sur une vitesse de rotation de 200 t/min.

On introduit alors exactement et en une seule fois 5 g de la poudre tamisée et on déclenche le chronomètre.

Lorsque la solubilisation est totale (aucune particule détectable à l'oeil), on stoppe le chronomètre et on relève le temps écoulé.

Dans ces conditions, la poudre obtenue à partir des granulés contenant des particules cristallines de xylitol et de l'autre polyol préparés conforme à l'invention a généralement une vitesse de solubilisation inférieure à la minute, de préférence comprise entre 20 et 30 ± 5 secondes.

Afin de mesurer la masse volumique apparente de la poudre obtenue à partir des granulés contenant des particules cristallines de xylitol et d'un autre polyol préparés conforme à l'invention, on réalise le test C.

Le test C est destiné à déterminer, dans des conditions définies, les volumes apparents avant et après tassement, l'aptitude au tassement, ainsi que les masses volumiques apparentes des poudres obtenues.

Pour les besoins du test C, la poudre obtenue à partir des granulés contenant des particules cristallines de xylitol et d'un autre polyol préparés conforme à l'invention est broyée et tamisée de manière à ce que soit obtenue une poudre présentent une taille de particules comprise entre 100 et 1.000 µm.

L'appareillage est un voluménomètre de tassage STAV 2003 commercialisé par J. ENGELSMANN AG qui est constitué par :
- un appareil de tassement pouvant provoquer par minute 250 chutes ± 15 chutes d'une hauteur de 3 mm ± 0,2 mm. Le support de l'éprouvette, avec son dispositif de fixation, doit avoir une masse de 450 g ± 5 g ;
- une éprouvette de 250 ml graduée tous les 2 ml, dont la masse doit être de 220 g ± 40 g.

Dans l'éprouvette sèche, on introduit sans tasser une prise d'essai M de 100 g pesée avec une précision de 0,5 %. On fixe l'éprouvette sur son support. On lit le volume apparent avant tassement V₀ estimé à 1 ml près.

On fait subir 10, 500 et 1.250 chutes et on lit les volumes correspondants V₁₀, V₅₀₀ et V₁₂₅₀, estimés à 1 ml près.

Les masses volumiques apparentes sont données par les expressions suivantes :
- masse volumique apparente avant tassement (dite masse volumique du produit vrac) = 100 / volume apparent avant tassement,
- masse volumique apparente après tassement (dite masse volumique du produit tassé) = 100 / volume apparent après 1.250 chutes.

Les résultats montrent que la poudre obtenue à partir des granulés contenant des particules cristallines de xylitol et de l'autre polyol préparés conforme à l'invention présente une valeur de masse volumique apparente tassée comprise entre 0,5 et 2 g/ml, de préférence comprise entre 0,6 et 1 g/ml.

La présente demande concerne également l'utilisation d'une poudre obtenue à partir des granulés préparés par un procédé selon l'invention, pour la fabrication de comprimés.

Les exemples suivants illustrent la préparation de la poudre à partir des granulés contenant des particules cristallines de xylitol et de l'autre polyol préparés par le procédé selon l'invention.

### Exemple 1

Une solution ayant une teneur de 95 % en xylitol et de 5 % en sorbitol, sur la base de la matière sèche, est placée dans un récipient d'évaporation pour obtenir un sirop de xylitol et de sorbitol ayant une teneur en matière sèche de 95 %.

Ce sirop de xylitol et de sorbitol est placé dans un réservoir de stockage à une température d'environ 80°C, à partir duquel il est continuellement prélevé au moyen d'une pompe qui assure sa dispersion sous forme de globules au moyen d'une buse.

Simultanément à la dispersion dudit sirop de xylitol et de sorbitol, on introduit continuellement du matériau de germes supplémentaire dans le granulateur à échelle pilote pour réaliser un rapport pondéral de germes/sirop d'environ 1 partie de germes pour 1 partie de sirop de xylitol et de sorbitol.

Les germes sont obtenus par le recyclage continu d'une fraction du matériau solidifié étant produit. Des particules d'un solide de xylitol cristallin quelconque peuvent être utilisées pour fournir des germes pour la granulation initiale.

Aucun effort particulier n'est entrepris pour contrôler la température du granulateur.

Le granulateur tourne à une vitesse d'environ 6,5 tours par minute, son inclinaison étant de 30°, ce qui permet d'obtenir des granulés ayant un diamètre moyen d'environ 500 *µ*m à 10.000 *µ*m.

Après cette étape de granulation, lesdits granulés sont maturés à 60°C par l'achèvement de la cristallisation dans un dispositif de maturation (tambour rotatif allongé).

Les granulés maturés ainsi obtenus présentent une composition de 97,3/2,7 en xylitol/sorbitol et sont séchés dans un lit fluidisé en utilisant de l'air à environ 45°C puis soumis à un broyage grossier.

Les granulés ainsi traités apparaissent sous la forme d'une poudre contenant environ 0,27 % d'humidité résiduelle.

La poudre séchée est ensuite tamisée. La vitesse de solubilisation, telle que mesurée selon le test A, est de 30 secondes, et la valeur de dureté déterminée selon le test B est de 130 N.

Le tableau I suivant présente les résultats des mesures effectuées selon le test C.

**Tableau I**

| | |
|---|---|
| 100 g de poudre utilisée Fraction 100 - 1.000 *µ*m | |
| Masse volumique apparente avant tassement (g/ml) | 0,588 |
| V₀ moyen (ml) sur 3 mesures | 170 |
| V₁₀ moyen (ml) sur 3 mesures | 162 |
| V₅₀₀ moyen (ml) sur 3 mesures | 150 |
| V_{1.250} moyen (ml) sur 3 mesures | 148 |

La masse volumique apparente du produit vrac est ainsi de 0,588 g/ml, et la masse volumique apparente du produit tassé de 0,676 g/ml.

### Exemple 2

Une solution ayant une teneur de 85 % en xylitol et de 15 % en sorbitol sur la base de la matière sèche est placée dans un récipient d'évaporation pour obtenir un sirop de xylitol et sorbitol ayant une teneur en matière sèche de 95 %.

Ce sirop de xylitol et de sorbitol est placé dans un réservoir de stockage à une température d'environ 80°C, à partir duquel il est continuellement prélevé au moyen d'une pompe qui assure sa dispersion sous forme de globules au moyen d'une buse.

Simultanément à la dispersion dudit sirop de xylitol et de sorbitol, on introduit continuellement du matériau de germes supplémentaire dans le granulateur à échelle pilote pour réaliser un rapport pondéral de germes/sirop d'environ 1 partie de germes pour 1 partie de sirop de xylitol et de sorbitol.

Les germes sont obtenus par le recyclage continu d'une fraction du matériau solidifié étant produit. Des particules d'un solide de xylitol cristallin quelconque peuvent être utilisées pour fournir des germes pour la granulation initiale.

Aucun effort particulier n'est entrepris pour contrôler la température du granulateur.

Le granulateur tourne à une vitesse d'environ 6,5 tours par minute, son inclinaison étant de 30°, ce gui permet d'obtenir des granulés ayant un diamètre moyen d'environ 500 *µ*m à 10.000 *µ*m.

Après cette étape de granulation, lesdits granulés sont maturés dans une étuve à 60°C pendant 30 à 40 minutes.

Les granulés maturés ainsi obtenus présentent une composition 90/10 en xylitol/sorbitol et sont séchés dans un lit fluidisé en utilisant de l'air à environ 45°C puis soumis à un broyage grossier.

Les granulés ainsi traités apparaissent sous la forme d'une poudre contenant environ 0,41 % d'humidité résiduelle.

La poudre séchée est ensuite tamisée. La vitesse de solubilisation, telle que mesurée selon le test A, est de 20 secondes, et la valeur de dureté déterminée selon le test B est de 170 N.

Le tableau II suivant présente les résultats des mesures effectuées selon le test C.

**Tableau II**

| | |
|---|---|
| 100 g de poudre utilisée Fraction 100 - 1.000 *µ*m | |
| Masse volumique apparente avant tassement (g/ml) | 0,645 |
| V₀ moyen (ml) sur 3 mesures | 155 |
| V₁₀ moyen (ml) sur 3 mesures | 150 |
| V₅₀₀ moyen (ml) sur 3 mesures | 137 |
| V_{1.250} moyen (ml) sur 3 mesures | 134 |

La masse volumique apparente du produit vrac est ainsi de 0,645 g/ml, et la masse volumique apparente du produit tassé de 0,746 g/ml.

### Exemple 3

Une solution ayant une teneur de 85 % en xylitol et de 15 % en maltitol sur la base de la matière sèche est placée dans un récipient d'évaporation pour obtenir un sirop de xylitol et de maltitol ayant une teneur en matière sèche de 95 %.

Ce sirop de xylitol et de maltitol est placé dans un réservoir de stockage à une température d'environ 80°C, à partir duquel il est continuellement prélevé au moyen d'une pompe qui assure sa dispersion sous forme de globules au moyen d'une buse.

Simultanément à la dispersion dudit sirop de xylitol et de maltitol, on introduit continuellement du matériau de germes supplémentaire dans le granulateur à échelle pilote pour réaliser un rapport pondéral de germes/sirop d'environ 1 partie de germes pour 1 partie de sirop de xylitol et de maltitol.

Les germes sont obtenus par le recyclage continu d'une fraction du matériau solidifié étant produit. Des particules d'un solide de xylitol cristallin quelconque peuvent être utilisées pour fournir des germes pour la granulation initiale.

Aucun effort particulier n'est entrepris pour contrôler la température du granulateur.

Le granulateur tourne à une vitesse d'environ 6,5 tours par minute, son inclinaison étant de 30°, ce qui permet d'obtenir des granulés ayant un diamètre moyen d'environ 500 *µ*m à 10.000 *µ*m.

Après cette étape de granulation, lesdits granulés sont maturés dans une étuve à 70°C pendant 30 à 40 minutes.

Les granulés maturés ainsi obtenus présentent une composition 90/10 en xylitol/maltitol et sont séchés dans un lit fluidisé en utilisant de l'air à environ 45°C, puis soumis à un broyage grossier.

Les granulés ainsi traités apparaissent sous la forme d'une poudre contenant environ 0,37 % d'humidité résiduelle.

La poudre séchée est ensuite tamisée. La vitesse de solubilisation, telle que mesurée selon le test A, est de 23 secondes, et la valeur de dureté déterminée selon le test B est de 95 N.

Le tableau III suivant présente les résultats des mesures effectuées selon le test C.

**Tableau III**

| | |
|---|---|
| 100 g de poudre utilisée Fraction 100 - 1.000 *µ*m | |
| Masse volumique apparente avant tassement (g/ml) | 0,794 |
| V₀ moyen (ml) sur 3 mesures | 126 |
| V₁₀ moyen (ml) sur 3 mesures | 118 |
| V₅₀₀ moyen (ml) sur 3 mesures | 108 |
| V_{1.250} moyen (ml) sur 3 mesures | 108 |

La masse volumique apparente du produit vrac est ainsi de 0,794 g/ml, et la masse volumique apparente du produit tassé de 0,926 g/ml.

## Revendications

1. Procédé de fabrication de granulés contenant des particules cristallines de xylitol et d'un autre polyol, comprenant le mélange continu d'un sirop de xylitol et d'un autre polyol, présentant une teneur en matière sèche d'au moins 95 % en poids, ayant une teneur en xylitol de 85 à 97,5 % en poids et en l'autre polyol de 15 à 2,5 % en poids sur la base de la matière sèche, ledit mélange étant effectué en dispersant simultanément le sirop de xylitol et de l'autre polyol avec des germes contenant du xylitol dans un récipient rotatif ouvert contenant des granulés à base de xylitol, ce par quoi le sirop de xylitol et de l'autre polyol et les germes contenant du xylitol sont mélangés à la surface des granulés à base de xylitol contenus dans le récipient, la récupération de granulés à base de xylitol et de l'autre polyol ayant un diamètre de 100 à 10.000 µm depuis le récipient, et la cristallisation du xylitol et de l'autre polyol contenus dans lesdits granulés, les granulés à base de xylitol et de l'autre polyol dans le récipient étant maintenus en mouvement par la rotation du récipient.

2. Procédé selon la revendication 1, dans lequel l'autre polyol est choisi dans le groupe constitué par le threitol, l'érythritol, l'arabitol, le ribitol, le sorbitol, le mannitol, le maltitol, le maltotriitol, le maltotétraitol, le lactitol, l'isomaltulose hydrogéné, la glycérine et les hydrolysats d'amidon hydrogénés, pris seuls ou en combinaison.

3. Procédé selon l'une ou l'autre des revendications 1 et 2 dans lequel l'autre polyol est de préférence choisi dans le groupe constitué par le sorbitol, le maltitol, le mannitol et l'isomaltulose hydrogéné, pris seuls ou en combinaison.

4. Procédé selon l'une quelconque des revendications 1 à 3, dans lequel le sirop de xylitol et de l'autre polyol est introduit dans le récipient sous forme subdivisée.

5. Procédé selon la revendication 4, dans lequel le sirop de xylitol et de l'autre polyol est introduit sous forme de gouttes.

6. Procédé selon la revendication 4, dans lequel le sirop de xylitol et de l'autre polyol est introduit sous forme de jets.

7. Procédé selon l'une quelconque des revendications 1 à 3, dans lequel l'axe de rotation du récipient est incliné par rapport à l'horizontale.

8. Procédé selon l'une quelconque des revendications 1 à 3, dans lequel les granulés à base de xylitol et de l'autre polyol sont récupérés par débordement a la sortie du récipient.

9. Procédé selon l'une quelconque des revendications 1 à 3, dans lequel un sirop de xylitol et de l'autre polyol, présentant une teneur en matière sèche de 95% en poids, est porté à une température d'au moins 80°C et est mélangé continuellement avec des germes contenant du xylitol, le rapport germes/sirop, les dimensions, l'orientation de l'axe de rotation et la vitesse de rotation du récipient étant choisis de telle façon que le produit récupéré depuis le récipient apparait sous la forme de granulés ayant un diamètre de 100 à 10.000 µm,

10. Procédé selon la revendication 9, dans lequel le rapport pondéral germes/sirop est de 1/1.

## Patentansprüche

1. Verfahren zur Herstellung von Granulat, welches Kristallpartikel von Xylit und einem anderen Polyol enthält und das ständige Mischen eines Sirups aus Xylit und einem anderen Polyol umfasst, mit einem Trockenmasseanteil von wenigstens 95 Gewichtsprozent, einem Xylit-Gehalt von 85 bis 97,5 Gewichtsprozent und einen Gehalt eines anderen Polyols von 15 bis 2,5 Gewichtsprozent basierend auf der Trockenmasse, wobei die Mischung durch gleichzeitiges Dispergieren des Sirups aus Xylit und dem anderen Polyol mit xylithaltigen Keimen in einem offenen, Drehbehälter erfolgt, der Granulat auf Xylitbasis enthält, wodurch der Sirup aus Xylit und dem anderen Polyol und die xylithaltigen Keime an der Oberfläche des in dem Behälter enthaltenen Granulats auf Xylitbasis gemischt werden, die Gewinnung von Granulat auf Basis von Xylit und dem anderen Polyol mit einem Durchmesser von 100 bis 10.000 µm aus dem Behälter, und die Kristallisation von in dem Granulat enthaltenen Xylit und dem anderen Polyol, wobei das Granulat auf Basis von Xylit und dem anderen Polyol in dem Behälter durch die Drehung des Behälters in Bewegung gehalten wird.

2. Verfahren nach Anspruch 1, wobei das andere Polyol aus der Gruppe gewählt ist, welche umfasst: Threitol, Erythritol, Arabitol, Ribitol, Sorbitol, Mannitol, Maltitol, Maltotriitol, Maltotetraitol, Lactitol, hydrierte Isomaltulose, Glycerin und Hydrolysate aus hydrierter Stärke, einzeln oder in Kombination.

3. Verfahren nach einem der Ansprüche 1 oder 2, bei welchem das andere Polyol bevorzugt aus der Gruppe gewählt ist, welche umfasst: Sorbitol, Maltitol, Mannitol und hydrierte Isomaltulose, einzeln oder in Kombination.

4. Verfahren nach einem der Ansprüche 1 bis 3, bei welchem der Sirup aus Xylit und dem anderen Polyol aufgeteilt in den Behälter hineingegeben wird.

5. Verfahren nach Anspruch 4, bei welchem der Sirup aus Xylit und dem anderen Polyol als Tropfen in den Behälter hineingegeben wird.

6. Verfahren nach Anspruch 4, bei welchem der Sirup aus Xylit und dem anderen Polyol als Strahl in den Behälter hineingegeben wird.

7. Verfahren nach einem der Ansprüche 1 bis 3, bei welchem die Drehachse des Behälters bezüglich der Horizontalen geneigt ist.

8. Verfahren nach einem der Ansprüche 1 bis 3, bei welchem das Granulat auf Basis von Xylit und dem anderen Polyol durch Überlauf am Ausgang des Behälters gewonnen wird.

9. Verfahren nach einem der Ansprüche 1 bis 3, bei welchem ein Sirup aus Xylit und dem anderen Polyol mit einem Trockenmassegehalt von 95 Gewichtsprozent auf eine Temperatur von wenigstens 80 °C gebracht wird und kontinuierlich mit xylithaltigen Keimen gemischt wird, wobei das Verhältnis Keime/Sirup, die Maße, die Ausrichtung der Drehachse und die Drehgeschwindigkeit des Behälters derart gewählt werden, dass das aus dem Behälter gewonnene Produkt als Granulat mit einem Durchmesser von 100 bis 10.000 µm vorliegt.

10. Verfahren nach Anspruch 9, bei welchem das Gewichtsverhältnis Keime/Sirup 1/1 ist.

## Claims

1. A method for manufacturing a powder containing crystalline particles of xylitol and of another polyol, comprising the continuous mixing of a syrup of xylitol and of another polyol, having a solids content of at least 95 wt %, having a xylitol content of 85 to 97.5 wt % and a content of the other polyol of at least 15 to 2.5 wt % based on the solids content, said mixing being carried out by simultaneously dispersing the syrup of xylitol and of the other polyol with seeds containing xylitol in an open rotary container that contains xylitol-based granules, with which the syrup of xylitol and of the other polyol and the seeds containing xylitol are mixed at the surface of the xylitol-based granules contained in the container, the recovery of the granules based on xylitol and on the other polyol from the container, and the crystallization of the xylitol and of the other polyol contained in said granules, the granules based on xylitol and on the other polyol in the container being kept moving by the rotation of the container.

2. The method as claimed in claim 1, wherein the other polyol is chosen from the group consisting of threitol, erythritol, arabitol, ribitol, sorbitol, mannitol, maltitol, maltotriitol, maltotetraitol, lactitol, hydrogenated isomaltulose, glycerol and hydrogenated starch hydrolysates, taken alone or in combination.

3. The method as claimed in claim 1 or 2, wherein the other polyol is preferably chosen from the group consisting of sorbitol, maltitol, mannitol and hydrogenated isomaltulose, taken alone or in combination.

4. The method as claimed in anyone of claims 1 to 3, wherein the syrup of xylitol and of the other polyol is introduced into the container in a subdivided form.

5. The method as claimed in claim 4, wherein the syrup of xylitol and of the other polyol is introduced in drop form.

6. The method as claimed in claim 4, wherein the syrup of xylitol and of the other polyol is introduced in the form of jets.

7. The method as claimed in anyone of claims 1 to 3, wherein the axis of rotation of the container is inclined relative to the horizontal.

8. The method as claimed in anyone of claims 1 to 3, wherein the granules based on xylitol and on the other polyol are recovered by overflowing at the outlet of the container.

9. The method as claimed in claim 1, wherein a syrup of xylitol and of the other polyol, having a solids content of 95 wt %, is brought to a temperature of at least 80[deg.] C. and is continually mixed with seeds containing xylitol, the seeds/syrup ratio, the dimensions, the orientation of the axis of rotation and the speed of rotation of the container being chosen such that the product recovered from the container appears in the form of granules having a diameter of 100 to 10 000 µm.

10. The method as claimed in claim 10, in which the seeds/syrup weight ratio is 1/1.
